(19) Europäisches Patentamt
European Patent Office
Office européen des brevets

(11) EP 3 270 923 B1

(12) **EUROPEAN PATENT SPECIFICATION**

(45) Date of publication and mention of the grant of the patent:
**26.05.2021 Bulletin 2021/21**

(21) Application number: **16765135.5**

(22) Date of filing: **18.03.2016**

(51) Int Cl.:
***A61K 31/4439*** *(2006.01)* ***A61P 25/00*** *(2006.01)*
***A61P 25/16*** *(2006.01)* ***A61P 25/28*** *(2006.01)*

(86) International application number:
**PCT/JP2016/059788**

(87) International publication number:
**WO 2016/148308 (22.09.2016 Gazette 2016/38)**

(54) **THERAPEUTIC AGENT FOR FRONTAL LOBE DYSFUNCTION**
THERAPEUTIKUM FÜR FRONTALLAPPENDYSFUNKTION
AGENT THÉRAPEUTIQUE POUR LE DYSFONCTIONNEMENT DU LOBE FRONTAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**MA MD**

(30) Priority: **19.03.2015 JP 2015055532**

(43) Date of publication of application:
**24.01.2018 Bulletin 2018/04**

(73) Proprietor: **Kyowa Kirin Co., Ltd.**
**Tokyo 100-0004 (JP)**

(72) Inventor: **HORITA, Takako**
**Tokyo 100-0004 (JP)**

(74) Representative: **Vossius & Partner**
**Patentanwälte Rechtsanwälte mbB**
**Siebertstrasse 3**
**81675 München (DE)**

(56) References cited:

**EP-A1- 1 700 856** **EP-A1- 1 700 856**
**EP-A1- 2 433 938** **EP-A1- 2 433 938**
**EP-A1- 2 474 543** **EP-A1- 2 474 544**
**WO-A1-2012/060844**

- **KADOWAKI HORITA TAKAKO ET AL: "Effects of the adenosine A2Aantagonist istradefylline on cognitive performance in rats with a 6-OHDA lesion in prefrontal cortex", PSYCHOPHARMACOLOGY, SPRINGER VERLAG, BERLIN, DE, vol. 230, no. 3, 10 June 2013 (2013-06-10) , pages 345-352, XP035370128, ISSN: 0033-3158, DOI: 10.1007/S00213-013-3158-X [retrieved on 2013-06-10]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 270 923 B1

## Description

Technical Field

The present invention relates to a therapeutic and/or prophylactic agent for use in frontal lobe dysfunction, wherein the frontal lobe dysfunction is cognitive impairment selected from the group consisting of cognitive impairment in Parkinson's disease (for example, executive dysfunction, memory disorder (particularly short term memory disorder), impairment in visual spatial cognition, smell disturbance and the like), cognitive impairment caused by chronic stress, dementia with Lewy bodies and frontotemporal dementia, or Lewy body disease (for example, cognitive impairment in Parkinson's disease, diffuse Lewy body disease, dementia with Lewy bodies, movement disorder associated with Lewy body disease and the like).

Background Art

The prefrontal cortex of the frontal lobe of the brain is responsible for cognitive functions (for example, comprehension, judgment, calculation ability, orientation, executive function and the like). Neurotransmitters such as dopamine, serotonin, norepinephrine, gamma-aminobutyric acid and the like are involved in the function of prefrontal cortex, and shortage-of these substances causes cognitive impairment. For example, it has been reported that decline in dopamine function and decline in cognitive function are correlated in elderly people (American Journal of Psychiatry 155:3, p.344 (1998)). It has also been reported that decline in dopamine function in the prefrontal cortex is possibly related to cognitive impairment in Parkinson's disease and cognitive impairment caused by chronic stress (Archives of Neurology, 57, p.470 (2000); The Journal of Neuroscience, 20(4), p.1568 (2000)). It has been reported that dopamine is deficient in the prefrontal cortex of Parkinson's disease patients with dementia and dementia with Lewy bodies patients (Neurology, 74, p.885 (2010)).

$\alpha$-Synuclein is a protein present in large amounts in the intracerebral presynaptic terminal, and involved in synaptic plasticity and neurotransmission (Journal of Chemical Neuroanatomy, 42, p.242 (2011)). $\alpha$-Synucleinopathy is a generic term for neurodegenerative diseases characterized by accumulation and formation of aggregation of $\alpha$-synuclein, and examples thereof comprise Lewy body diseases (for example, Parkinson's disease dementia, diffuse Lewy body disease, dementia with Lewy bodies, movement disorder associated with Lewy body disease and the like), multiple system atrophy (for example, olivopontocerebellar atrophy, striatonigral degeneration, Shy-Drager syndrome and the like) and the like.

In Lewy body disease, Lewy bodies containing $\alpha$-synuclein aggregates as a main component are found in nerve cells and, in multiple system atrophy, $\alpha$-synuclein-positive inclusions body are found in glial cells. Various symptoms such as parkinsonism, cognitive impairment, autonomic symptom, cerebellar ataxia and the like manifest themselves depending on the level of distribution of these pathologies (Parkinsonism and related disorders, 20S1, p.S62 (2014)).

The cognitive impairment in Lewy body diseases is considered to involve dopaminergic and cholinergic hypofunctions in the frontal lobe (Brain, 137, p.2493 (2014); Neurology, 74, p.885 (2010)). The prefrontal cortex of the frontal lobe in the brain is a region responsible for cognitive functions (for example, comprehension, judgment, calculation ability, orientation, executive function and the like). Neurotransmitters such as dopamine, serotonin, norepinephrine, gamma-aminobutyric acid and the like are involved in the function of prefrontal cortex, and shortage of these substances causes cognitive impairment.

On the other hand, as regards the relationship between adenosine $A_{2A}$ receptor and cognitive function, it is known that adenosine $A_{2A}$ receptor deficient mice show increased working memory (non-patent document 1). A triazolotriazine derivative having an adenosine $A_{2A}$ receptor antagonistic activity is known to improve short-term social memory disorders in hypertension rats (non-patent document 2). Furthermore, an adenosine $A_{2A}$ receptor antagonist, istradefylline, is known to have a suppressive activity on neurodegeneration (for example, patent document 1), an improving effect on cognitive impairment in Parkinson's disease (non-patent document 3) and the like.

A compound represented by the formula (I) is known to have affinity for adenosine $A_{2A}$ receptors and has a therapeutic effect on Parkinson's disease (patent document 2). Also, this compound is known to be useful as a therapeutic and/or prophylactic agent for movement disorders (patent document 3). Patent document 4 relates to pharmaceutical combinations comprising an $A_{2a}$ antagonist which are reported to act as cognition and motor fluctuation enhancers.

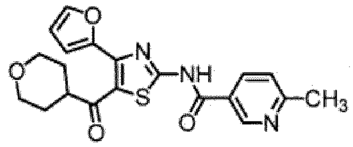

( I )

[Prior Art Documents]

patent documents

[patent document 1] WO 99/12546
[patent document 2] WO 2005/063743
[patent document 3] WO 2010/126082
[patent document 4] WO 2012/060844 A1

non-patent documents

[non-patent document 1] "Brain Research", 2009, vol. 1303, p.74
[non-patent document 2] "Behavioural Brain Research", 2005, vol. 159, p.197
[non-patent document 3] "Psychopharmacology", 2013, vol. 230, p.345

SUMMARY OF THE INVENTION

Problems to be Solved by the Invention

An object of the present invention is to provide a therapeutic and/or prophylactic agent for use in frontal lobe dysfuntion, wherein the frontal lobe dysfunction is cognitive impairment selected from the group consisting of cognitive impairment in Parkinson's disease (for example, executive dysfunction, memory disorder (particularly short term memory disorder), impairment in visual spatial cognition, smell disturbance and the like), cognitive impairment caused by chronic stress, dementia with Lewy bodies and frontotemporal dementia, or Lewy body disease (for example, cognitive impairment in Parkinson's disease, diffuse Lewy body disease, dementia with Lewy bodies, movement disorder associated with Lewy body disease and the like).

Means of Solving the Problems

The present invention relates to the following items:

1. A compound represented by the formula (I):

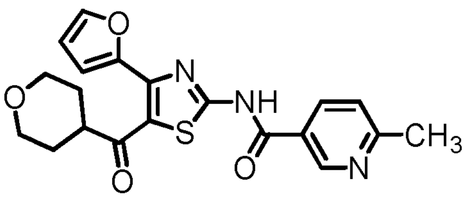

( I )

or a pharmaceutically acceptable salt thereof, for use in the treatment and/or prophylaxis of frontal lobe dysfunction, wherein the frontal lobe dysfunction is

(1) cognitive impairment selected from the group consisting of cognitive impairment in Parkinson's disease,

cognitive impairment caused by chronic stress, dementia with Lewy bodies and frontotemporal dementia; or
(2) cognitive impairment due to a decline in dopamine function in the medial prefrontal cortex.

2. The compound or the pharmaceutically acceptable salt thereof for use according to item 1, wherein the cognitive impairment is cognitive impairment in Parkinson's disease.

3. The compound or the pharmaceutically acceptable salt thereof for use according to item 2, wherein the cognitive impairment in Parkinson's disease is executive dysfunction, memory disorder, impairment in visual spatial cognition or smell disturbance.

4. The compound or the pharmaceutically acceptable salt thereof for use according to item 1, wherein the cognitive impairment is cognitive impairment caused by chronic stress.

5. The compound or the pharmaceutically acceptable salt thereof for use according to item 1, wherein the cognitive impairment is dementia with Lewy bodies.

6. A compound represented by the formula (I):

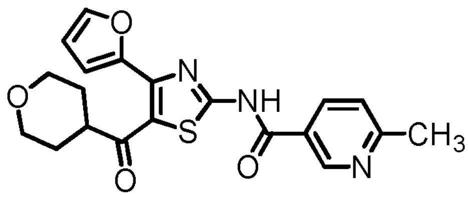

(I)

or a pharmaceutically acceptable salt thereof, for use in the treatment and/or prophylaxis of Lewy body disease.

7. The compound or the pharmaceutically acceptable salt thereof for use according to item 6, wherein the Lewy body disease is cognitive impairment in Parkinson's disease.

8. The compound or the pharmaceutically acceptable salt thereof for use according to item 6, wherein the Lewy body disease is diffuse Lewy body disease.

9. The compound or the pharmaceutically acceptable salt thereof for use according to item 6, wherein the Lewy body disease is dementia with Lewy bodies.

10. The compound or the pharmaceutically acceptable salt thereof for use according to item 6, wherein the Lewy body disease is movement disorder associated with Lewy body disease. >>

Effect of the Invention

According to the present invention, a therapeutic and/or prophylactic agent and the like for frontal lobe dysfunction, wherein the frontal lobe dysfunction is cognitive impairment selected from the group consisting of cognitive impairment in Parkinson's disease (for example, executive dysfunction, memory disorder (particularly short term memory disorder), impairment in visual spatial cognition, smell disturbance and the like), dementia with Lewy bodies, cognitive impairment caused by chronic stress and frontotemporal dementia, or Lewy body disease (for example, cognitive impairment in Parkinson's disease, diffuse Lewy body disease, dementia with Lewy bodies, movement disorder associated with Lewy body disease, which comprises a compound represented by the formula (I):

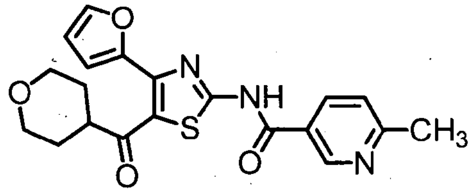

(I)

or a pharmaceutically acceptable salt thereof, as an active ingredient are provided.

The therapeutic and/or prophylactic agent of the present invention can be utilized for the treatment and/or prophylaxis of, for example, diseases such as frontal lobe dysfunction, among others, cognitive impairment due to a decline in dopamine function in the medial prefrontal cortex. Mode for Carrying out the Invention

Examples of the frontal lobe dysfunction in the present invention include cognitive impairment in Parkinson's disease (for example, executive dysfunction, memory disorder (particularly short term memory disorder), impairment in visual spatial cognition, smell disturbance and the like), cognitive impairment caused by chronic stress, dementia with Lewy bodies and frontotemporal dementia.

These diseases are related to a decline in dopamine function in the medial prefrontal cortex.

Examples of the Lewy body disease in the present invention include cognitive impairment in Parkinson's disease, diffuse Lewy body disease, dementia with Lewy bodies, movement disorder associated with Lewy body disease and the like.

In the following, a compound represented by the formula (I) is sometimes to be referred to as compound (I).

A pharmaceutically acceptable salt of compound (I) encompasses, for example, a pharmaceutically acceptable acid addition salt, a metal salt, an ammonium salt, an organic amine addition salt, an amino acid addition salt and the like. Examples of the pharmaceutically acceptable acid addition salt of compound (I) include inorganic acid salts such as hydrochloride, hydrobromide, nitrate, sulfate, phosphate and the like, organic acid salts such as acetate, oxalate, maleate, fumarate, citrate, benzoate, methanesulfonate and the like, and the like. Examples of the pharmaceutically acceptable metal salt include alkali metal salts such as sodium salt, potassium salt and the like, alkaline earth metal salts such as magnesium salt, calcium salt and the like, aluminum salt, zinc salt and the like. Examples of the pharmaceutically acceptable ammonium salt include salts of ammonium, tetramethylammonium and the like, examples of the pharmaceutically acceptable organic amine addition salt include addition salts with morpholine, piperidine and the like, and examples of the pharmaceutically acceptable amino acid addition salt include addition salts of lysine, glycine, phenylalanine, aspartic acid, glutamic acid and the like.

Compound (I) or the pharmaceutically acceptable salts thereof to be used in the present invention can be produced, for example, by the method described in Example 504 of WO 2005/063743.

When a salt of compound (I) is desired and compound (I) is obtained in the form of a salt, it only needs to be directly purified, or when it is obtained as a free form, compound (I) is dissolved or suspended in a suitable solvent, and an acid or base is added to form a salt, which is isolated and purified.

Also, compound (I) or a pharmaceutically acceptable salt thereof may be present in the form of an adduct with water or various solvents, and such adduct can also be used as the therapeutic and/or prophylactic agent of the present invention.

Next, the representative pharmacological effect of compound (I) is concretely explained by way of Experimental Examples.

[Experimental Example 1] Effect of compound (I) in object recognition test using medial prefrontal dopaminergic terminal-lesioned rat

The object recognition test is known as an evaluation system of cognitive function utilizing properties of animal to willingly explore a new object (Behavioural Brain Research 31, p.47 (1988)). An animal is placed in an apparatus containing two identical objects and allowed to freely explore (acquisition trial), after which one of the objects in the apparatus is changed to one having a different shape (novel object) and the animal is allowed to freely explore (test trial). When the animal remembers the shape of the object presented in the acquisition trial, it shows the property to explore the novel object for a longer time. In this test, such property is evaluated as an index of cognitive function. The medial prefrontal dopaminergic terminal-lesioned rat.used in this test shows a decrease in the dopamine content and basal level of dopamine release of the medial prefrontal cortex, as well as working memory disorders, and is considered a model reflecting a decline in the function of prefrontal cortex (Psychopharmacology, 230, p. 345 (2013)).

<Preparation of animal model>

CD(SD)IGS rat (male, Charles River) received Pentobarbital sodium salt (Tokyo Chemical Industry Co., Ltd., 30 mg/kg) intraperitoneally and fixed on a brain stereotaxis apparatus (SR-6, Narishige kagaku kikai kenkyusho) under anesthesia. Using a microinjection pump (CMA/100, Carnegie Medicine), dopaminergic neurotoxin, 6-hydroxidopamine hydrochloride (6-OHDA, Sigma-Aldrich) was injected over about 1 min at (1) 3.2 mm anterior, 0.8 mm left lateral and 3.0 mm ventral, (2) 3.2 mm anterior, 0.8 mm right lateral and 3.0 mm ventral, (3) 3.2 mm anterior, 0.8 mm left lateral and 5.0 mm ventral, (4) 3.2 mm anterior, 0.8 mm right lateral and 5.0 mm ventral, (5) 4.2 mm anterior, 0.8 mm left lateral and 4.0 mm ventral, and (6) 4.2 mm anterior, 0.8 mm right lateral and 4.0 mm ventral, each from the bregma suture of the rats to lesion dopaminergic terminals in the medial prefrontal cortex. 6-OHDA was prepared at a concentration of 4 μg/μL, injected at a flow rate of 1 μL/min over 1 min, and stood for 1 min. In the sham operated group, a stainless cannula was inserted into the same coordinates. In this case, to prevent lesion of noradrenergic neurons, desipramine hydrochloride (Sigma-Aldrich, 25 mg/kg) was intraperitoneally administered about 30 min before 6-OHDA injection. The dosing volumes of pentobarbital sodium and desipramine hydrochloride were calculated at 1 and 5 mL/kg based on the body weight measured on the administration day. The rats were used for the efficacy study after a recovery period of not less than 5 days postsurgery.

<Object recognition test>

A yellow white apparatus with a circular shape (diameter 82 cm, height 20 cm) was used. As the object, a brown glass bottle (diameter 5 cm, height 8 cm) and a gray agate mortar (diameter 8.5 cm, height 5 cm) were used. The two objects were placed in the apparatus at 20 cm from the wall. The apparatus and objects were used for each trial after cleaning with 50% ethanol.

To familiarize the rats with the experimenter, they were handled for 3 days before the test. At 60 min before the test, compound (I) (suspended in 0.5 w/v% aqueous methylcellulose 400 (MC) solution and prepared to 0.5 mL per 100 g body weight of rats on the administration day) was orally administered at a dose of 0.3 mg/kg, or a vehicle (0.5 w/v% aqueous MC solution) free of the test compound was orally administered at 0.5 mL per 100 g body weight of rats on the administration day. Two identical objects were set in the apparatus, and the rats were placed therein, allowed to freely explore for 10 min (acquisition trial), and placed back in the home cage. Three minutes later, an object having the same shape as that of the object presented in the acquisition trial (familiar object) and an object having a different shape (novel object) were placed in the apparatus, and the rats were placed in the apparatus and allowed to freely explore for 3 min (test trial). The time spent in exploration (sniffing) each object of the rats in the test trial was measured, and the ratio (%) relative to the total exploration time was calculated. For data analysis, only the data of the rats having the total exploration time of not less than 5 seconds in the test trial were used.

<Results>

Table 1 shows the ratio of the familiar object and novel object exploration times relative to the total exploration time in mean ± standard error. For statistical analysis, statistical analysis software SAS (Release 9.1.3, SAS Institute Inc.) was used. For comparison of the exploration time of the familiar object and the novel object, paired t-test was performed after confirmation of the normal distribution by a Shapiro-Wilk test. The level of statistical significance was set at $p<0.05$.

Table 1

| Effect of compound (I) on exploration time | | | | |
|---|---|---|---|---|
| group | surgery/administration | ratio (%) of exploration time | | animal number (rats) |
| | | familiar object | novel object | |
| sham operated | sham operated /vehicle | 31.76 ± 3.98 | 68.24 ± 3.98 | 10 |
| vehicle administration | lesion/vehicle | 49.12 ± 3.53 | 50.88 ± 3.53 | 10 |
| compound (I) administration | lesion/compound (I) | 34.97 ± 2.88 | 65.03 ± 2.88 | 10 |

In Table, "lesion" means that surgery to lesion of dopaminergic terminals in the medial prefrontal cortex was performed.

In the sham operated group, the exploration time of the novel object was significantly longer than that of the familiar object ($p<0.01$). In the vehicle administration group, the exploration time was not different between them. In

contrast, in the compound (I) administration group, the exploration time of the novel object was significantly longer than that of the familiar object ($p<0.001$).

By the above-mentioned test, compound (I) was confirmed to improve cognitive impairment due to a decline in dopamine function in the medial prefrontal cortex.

That is, compound (I) or a pharmaceutically acceptable salt thereof is considered to be useful for the treatment and/or prophylaxis of frontal lobe dysfunction, wherein the frontal lobe dysfunction is cognitive impairment selected from the group consisting of cognitive impairment in Parkinson's disease (for example, executive dysfunction, memory disorder (particularly short term memory disorder), impairment in visual spatial cognition, smell disturbance and the like), cognitive impairment caused by chronic stress, dementia with Lewy bodies and frontotemporal dementia.

[Experimental Example 2] Effect of compound (I) in an α-synucleinopathy model

By reference to articles (Science, 338, p.949 (2012); Behavioral Brain Reexplore, 208, p.274 (2010)), an animal model capable of confirming a treatment and/or prophylactic effect on α-synucleinopathy, for example, Lewy body disease (for example, cognitive impairment in Parkinson's disease, diffuse Lewy body disease, dementia with Lewy bodies, movement disorder associated with Lewy body disease and the like) is generated.

An appropriate dose of a solution of α-synuclein (rPeptide, S-100) or NAC61-95 (custom, Sigma) dissolved in phosphate buffered saline (PBS) is injected to the striatum or lateral cerebral ventricle of SLC: ICR male mice to induce cognitive impairment and/or movement disorder. After confirmation of the pathology induction, an appropriate dose of compound (I) is administered to the mice. Improvement of the pathology of the mice in a few hours after the administration is confirmed by behavioral pharmacological evaluation such as Y-maze, amount of spontaneous motor activity, CATWALK and the like.

From the above-mentioned test, compound (I) can be confirmed to have a treatment and/or prophylactic effect on α-synucleinopathy, for example, Lewy body disease (for example, cognitive impairment in Parkinson's disease, diffuse Lewy body disease, dementia with Lewy bodies, movement disorder associated with Lewy body disease and the like) and the like.

[Experimental Example 3] Effect of compound (I) in an α-synucleinopathy model

By reference to articles (Science, 338, p.949 (2012); Behavioral Brain Reexplore, 208, p.274 (2010)), an animal model capable of confirming a treatment and/or prophylactic effect on α-synucleinopathy, for example, Lewy body disease (for example, cognitive impairment in Parkinson's disease, diffuse Lewy body disease, dementia with Lewy bodies, movement disorder associated with Lewy body disease and the like) was generated as follows.

The cognitive function was evaluated using a spontaneous alternation task. The spontaneous alternation task is known as an evaluation system of cognitive function utilizing properties of animal to willingly explore a novel environment (Neuroscience and Biobehavioral Reviews 28, p. 497 (2004)). That is, if the animal remembers the arm previously entered in exploration in a Y-maze apparatus, the behavior of spontaneous entry into a different arm is expressed by an alternation behavior rate, and used as an index of cognitive function. The motor function was evaluated by analyzing gait function in natural walk conditions using a gait analysis system.

<Preparation of animal model>

NAC61-95 (custom, Sigma) was dissolved in PBS to prepare 4 μg/μL NAC61-95 solution.

Under pentobarbital (Somnopentyl, Kyoritsuseiyaku, 50 mg/kg, i.p.) anesthesia, 5 μL of NAC61-95 solution was injected using a Hamilton syringe (10 μL) equipped with a double needle (27G, needle length 3 mm) into the right lateral cerebral ventricle of Slc:ICR mice (male, Japan SLC) over about 1 min, and stood for 1 min to induce cognitive impairment and/or movement disorder. The sham treatment group was injected with 5 μL of PBS.

<Spontaneous alternation task: evaluation of cognitive function>

Y-maze apparatus wherein three arms made of a black acrylic wall (height 20 cm, length 25 cm, width 5 cm) are each connected at 120 degree angle was used.

Mice were brought into the experiment room on the previous day of test for habituation. Mice were placed at the tip of any of the arms of the Y-maze apparatus and allowed to freely explore in the maze for 7 min. Entry of all four limbs of a mouse into one arm was defined as entry into arm, and the order of entry of the mice into the arm was recorded. A behavior of continuous entry into all three different arms was defined as spontaneous alternation behavior, and the percentage of alternation behavior was calculated by the following calculation formula.

$$\text{Alternation behavior (\%)} = \frac{\text{number of spontaneous alternation behaviors}}{(\text{total arm entries}) - 2} \times 100$$

For calculation of the alternation behavior, only the data of mice with not less than 10 total arm entries was used.

<Gait analysis: evaluation of motor function>

A gait analysis system (CatWalk XT, ver.9.1, Noldus) was used. The system was constituted of a walkway with pressure dependent luminescence glass and a light source, luminescence ceiling, highly sensitive highspeed camera and analysis software, and the gait was analyzed by digitizing the brightness of the light emitted by the pressure dependent luminescence glass in response to the pressure. The gait data was automatically recorded and analyzed by the analysis software only when the mice moved straight forward without stopping in the predetermined area on the walkway.

The mice were brought into the experiment room without light and habituated for not less than 1 hr. The mice were placed in the apparatus, and allowed to walk freely until 6 running data were obtained for each mouse. The earliest three analyzable running data were used, and mean was calculated. By reference to an article relating to movement disorder in Parkinson's disease animal model (Journal of Biomedical Science 17, p. 9 (2010)), a decrease in the maximum contact area (Total surface area of certain paw at the moment of maximum paw contact) and print area (total surface area of the paw print), as well as change in the gait pattern (sequence of contact of 4 paws) were used as indices of movement disorder.

<Drug treatment>

At 60 min before the test, compound (I) (suspended in 0.5 w/v% aqueous MC solution, and prepared to 0.5 mL per 100 g body weight of mouse on the administration day) was orally administered at a dose of 0.1 mg/kg, or a vehicle (0.5 w/v% aqueous MC solution) free of the test compound was orally administered at 0.5 mL per 100 g body weight of mice on the administration day.

<Results>

Table 2 shows the alternation behavior in the spontaneous alternation task in mean ± standard error. For statistical analysis, statistical analysis software SAS (Release 9.2, SAS Institute Inc.) was used. For comparison of the two groups, homoscedasticity was assumed from the results of F-test, and Student's t-test was performed. The level of statistical significance was set at $p<0.05$.

Table 2

| Effect of compound (I) on alternation behavior | | | |
|---|---|---|---|
| group | treatment/administration | alternation behavior (%) | animal number (mice) |
| sham treatment | vehicle/vehicle | 66.5 ± 1.6 | 16 |
| vehicle administration | NAC61-95/vehicle | 59.6 ± 1.6 | 16 |
| compound (I) administration | NAC61-95/compound (I) | 69.5 ± 2.4 | 16 |

The vehicle administration group showed a significantly low alternation behavior as compared to the sham treatment group ($p<0.01$), and cognitive impairment was induced by NAC61-95 treatment. In contrast, the compound (I) administration group showed a significantly high alternation behavior as compared to the vehicle administration group ($p<0.01$), and improvement of cognitive impairment by NAC61-95 treatment was found.
Table 3 shows each gait parameter in the gait test in mean ± standard error.

Table 3

| Effect of compound (I) on gait parameters | | | |
|---|---|---|---|
| Group | sham treatment | vehicle administration | compound (I) administration |
| treatment/ administration | vehicle/ vehicle | NAC61-95/vehicle | NAC61-95/ compound (I) |

(continued)

| Effect of compound (I) on gait parameters | | | | |
|---|---|---|---|---|
| Group | | sham treatment | vehicle administration | compound (I) administration |
| animal number (mice) | | 16 | 16 | 16 |
| maximum contact area | right forepaw | 0.32 ± 0.03 | 0.24 ± 0.02 | 0.29 ± 0.02 |
| | right hindpaw | 0.33 ± 0.03 | 0.27 ± 0.02 | 0.30 ± 0.02 |
| | Left forepaw | 0.31 ± 0.02 | 0.26 ± 0.02 | 0.30 ± 0.02 |
| | Left hindpaw | 0.31 ± 0.02 | 0.24 ± 0.02 | 0.31 ± 0.02 |
| print area | Right forepaw | 0.43 ± 0.04 | 0.33 ± 0.02 | 0.39 ± 0.02 |
| | Right hindpaw | 0.41 ± 0.03 | 0.34 ± 0.03 | 0.37 ± 0.03 |
| | left 1 forepaw | 0.42 ± 0.03 | 0.36 ± 0.03 | 0.40 ± 0.02 |
| | left hindpaw | 0.39 ± 0.03 | 0.29 ± 0.03 | 0.38 ± 0.03 |
| gait pattern | AA | 0.00 ± 0.00 | 2.79 ± 2.36 | 0.00 ± 0.00 |
| | AB | 89.91 ± 3.36 | 74.11 ± 8.85 | 81.86 ± 4.25 |
| | CA | 4.18 ± 2.10 | 5.84 ± 2.45 | 13.39 ± 4.00 |
| | CB | 5.91 ± 2.30 | 17.26 ± 6.92 | 4.75 ± 1.79 |
| AA: right forepaw →right hindpaw →left forepaw →left hindpaw<br>AB: right forepaw →left hindpaw →left forepaw →right hindpaw<br>CA: right forepaw →left forepaw →right hindpaw →left hindpaw<br>CB: right forepaw →left hindpaw →right hindpaw →left forepaw | | | | |

The vehicle administration group showed a significantly small maximum contact area and gait area of the right forepaw and left hindpaw as compared to the sham treatment group (both p<0.05), change in the gait pattern (decrease in pattern AB, increase in pattern CB), and movement disorder was induced by NAC61-95 treatment. In contrast, the compound (I) administration group showed a significantly large maximum contact area and gait area of the left hindpaw as compared to the vehicle administration group (p<0.05), tendency of the maximum contact area and gait area of the right forepaw being large (p<0.1), and the gait pattern with an increase in pattern AB and a decrease in pattern CB. From the above, improvement of movement disorder induced by NAC61-95 treatment was found in the compound (I) administration group.

From the above-mentioned test, compound (I) could be confirmed to have a treatment and/or prophylactic effect on α-synucleinopathy, for example, Lewy body disease (for example, cognitive impairment in Parkinson's disease, diffuse Lewy body disease, dementia with Lewy bodies, movement disorder associated with Lewy body disease and the like) and the like.

While compound (I) or a pharmaceutically acceptable salt thereof can be directly administered singly, it is generally desirably provided as various pharmaceutical preparations. Such pharmaceutical preparations are used for animals or human.

The pharmaceutical preparation of the present invention can contain compound (I) or a pharmaceutically acceptable salt thereof singly as an active ingredient or as a mixture with any other active ingredients. In addition, such pharmaceutical preparations are produced by mixing the active ingredient with one or more kinds of pharmaceutically acceptable carriers (for example, diluent, solvent, excipient and the like), and according to any method well known in the technical field of drug formulation study.

As an administration route, a route most effective for the treatment is desirably used, which may be oral or parenteral, for example, intravenous, transdermal and the like.

Examples of the administration form include tablet, injection, external preparation and the like.

A form suitable for oral administration, for example, tablet and the like, can be produced using excipient such as lactose and the like, disintegrant such as starch and the like, lubricant such as magnesium stearate and the like, binder such as hydroxypropylcellulose and the like, and the like.

A form suitable for parenteral administration, for example, injection and the like can be produced using a diluent or solvent such as salt solution, glucose solution, a mixture of salt water and glucose solution, and the like, and the like.

While a dosage form suitable for external preparation is not particularly limited, for example, ointment, cream, liniment, lotion, cataplasm, plaster, tape and the like can be mentioned. For example, ointment, cream and the like can be produced by, for example, dissolving or mixing and dispersing the active ingredient in a base such as white petrolatum and the like.

The dose and administration frequency of compound (I) or a pharmaceutically acceptable salt thereof vary depending on the administration form, age and body weight of patients, nature or severity of the symptom to be treated and the like. In the case of oral route, 0.01 - 1000 mg, preferably 0.05 - 100 mg, is generally administered to an adult once to several times per day. In the case of parenteral administration such as intravenous administration and the like, 0.001 - 1000 mg, preferably 0.01 - 100 mg, is generally administered to an adult once to several times per day. In the case of transdermal administration, an external preparation containing 0.001 - 10% of compound (I) or a pharmaceutically acceptable salt thereof is generally administered by applying once to several times. However, such dose and administration frequency vary depending on the aforementioned various conditions.

The present invention is explained in more detail in the following by referring to Examples, which are not to be construed as limitative.

## Examples

### [Example 1]

A tablet having the following composition is prepared by a conventional method. Compound (I) (40 g), lactose (286.8 g) and potatostarch (60 g) are mixed, and 10% aqueous solution (120 g) of hydroxypropylcellulose is added thereto. The mixture is kneaded, granulated, dried, and sieved to give granules for tableting by a conventional method. The granules are mixed with magnesium stearate (1.2 g) and the mixture is tableted by a tableting machine with a 8 mm punch (manufactured by Kikusui, RT-15) to give tablets (containing 20 mg of active ingredient per tablet).

Table 4

| Formulation | |
|---|---|
| compound (I) | 20 mg |
| lactose | 143.4 mg |
| potato starch | 30 mg |
| hydroxypropylcellulose | 6 mg |
| magnesium stearate | 0.6 mg |
| | 200 mg |

### [Example 2]

An injection having the following composition is prepared by a conventional method. Compound (I) (1 g) is mixed with injectable distilled water, pH is adjusted to 7 by adding hydrochloric acid and aqueous sodium hydroxide solution, and injectable distilled water is added to make the total amount 1000 mL. The obtained mixture is aseptically filled in a glass vial by 2 mL to give injections (containing 2 mg of active ingredient per vial).

Table 5

| Formulation | |
|---|---|
| compound (I) | 2 mg |
| hydrochloric acid | q.s. |
| aqueous sodium hydroxide solution | q.s. |
| injectable distilled water | q.s. |
| | 2.00 mL |

### [Reference Example 1]

Compound (I) was obtained according to the method described in Example 504 of WO 2005/063743.

Industrial Applicability

The present invention can be utilized for the treatment and/or prophylaxis of frontal lobe dysfunction, wherein the frontal lobe dysfunction is cognitive impairment selected from the group consisting of cognitive impairment in Parkinson's disease (for example, executive dysfunction, memory disorder (particularly short term memory disorder), impairment in visual spatial cognition, smell disturbance and the like), cognitive impairment caused by chronic stress, dementia with Lewy bodies, and frontotemporal dementia, or Lewy body disease (for example, cognitive impairment in Parkinson's disease, diffuse Lewy body disease, dementia with Lewy bodies, movement disorder associated with Lewy body disease and the like).

## Claims

1. A compound represented by the formula (I):

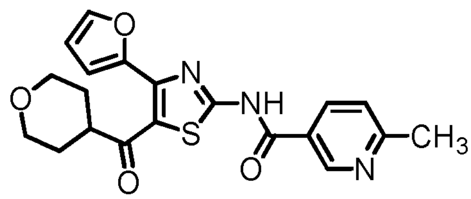

(I)

or a pharmaceutically acceptable salt thereof, for use in the treatment and/or prophylaxis of frontal lobe dysfunction, wherein the frontal lobe dysfunction is

(1) cognitive impairment selected from the group consisting of cognitive impairment in Parkinson's disease, cognitive impairment caused by chronic stress, dementia with Lewy bodies and frontotemporal dementia; or
(2) cognitive impairment due to a decline in dopamine function in the medial prefrontal cortex.

2. The compound or the pharmaceutically acceptable salt thereof for use according to claim 1, wherein the cognitive impairment is cognitive impairment in Parkinson's disease.

3. The compound or the pharmaceutically acceptable salt thereof for use according to claim 2, wherein the cognitive impairment in Parkinson's disease is executive dysfunction, memory disorder, impairment in visual spatial cognition or smell disturbance.

4. The compound or the pharmaceutically acceptable salt thereof for use according to claim 1, wherein the cognitive impairment is cognitive impairment caused by chronic stress.

5. The compound or the pharmaceutically acceptable salt thereof for use according to claim 1, wherein the cognitive impairment is dementia with Lewy bodies.

6. A compound represented by the formula (I):

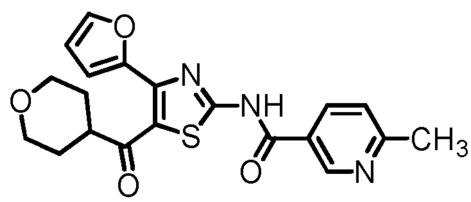

(I)

or a pharmaceutically acceptable salt thereof, for use in the treatment and/or prophylaxis of Lewy body disease.

7. The compound or the pharmaceutically acceptable salt thereof for use according to claim 6, wherein the Lewy body disease is cognitive impairment in Parkinson's disease.

8. The compound or the pharmaceutically acceptable salt thereof for use according to claim 6, wherein the Lewy body disease is diffuse Lewy body disease.

9. The compound or the pharmaceutically acceptable salt thereof for use according to claim 6, wherein the Lewy body disease is dementia with Lewy bodies.

10. The compound or the pharmaceutically acceptable salt thereof for use according to claim 6, wherein the Lewy body disease is movement disorder associated with Lewy body disease .

**Patentansprüche**

1. Eine Verbindung, dargestellt durch die Formel (I):

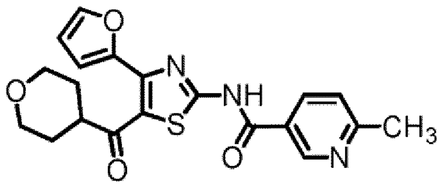

(I)

oder ein pharmazeutisch verträgliches Salz davon zur Verwendung bei der Behandlung und/oder Prophylaxe von Frontallappendysfunktion, wobei die Frontallappendysfunktion

(1) kognitive Beeinträchtigung, ausgewählt aus der Gruppe bestehend aus kognitiver Beeinträchtigung bei Morbus Parkinson, durch chronischen Stress verursachter kognitiver Beeinträchtigung, Demenz mit Lewy-Körperchen und frontotemporaler Demenz; oder
(2) kognitive Beeinträchtigung aufgrund einer Abnahme der Dopaminfunktion im medialen präfrontalen Kortex

ist.

2. Die Verbindung oder das pharmazeutisch verträgliche Salz davon zur Verwendung nach Anspruch 1, wobei die kognitive Beeinträchtigung kognitive Beeinträchtigung bei Morbus Parkinson ist.

3. Die Verbindung oder das pharmazeutisch verträgliche Salz davon zur Verwendung nach Anspruch 2, wobei die kognitive Beeinträchtigung bei Morbus Parkinson exekutive Dysfunktion, Gedächtnisstörung, Beeinträchtigung der visuellen räumlichen Wahrnehmung oder Beeinträchtigung des Geruchssinns ist.

4. Die Verbindung oder das pharmazeutisch verträgliche Salz davon zur Verwendung nach Anspruch 1, wobei die kognitive Beeinträchtigung durch chronischen Stress verursachte kognitive Beeinträchtigung ist.

5. Die Verbindung oder das pharmazeutisch verträgliche Salz davon zur Verwendung nach Anspruch 1, wobei die kognitive Beeinträchtigung Demenz mit Lewy-Körperchen ist.

6. Eine Verbindung, dargestellt durch die Formel (I):

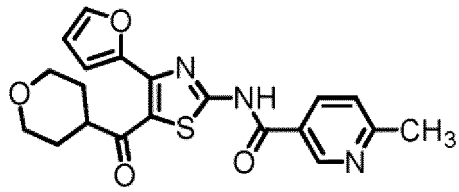

(I)

oder ein pharmazeutisch verträgliches Salz davon zur Verwendung bei der Behandlung und/oder Prophylaxe von Lewy-Körperchen-Krankheit.

7. Die Verbindung oder das pharmazeutisch verträgliche Salz davon zur Verwendung nach Anspruch 6, wobei die Lewy-Körperchen-Krankheit kognitive Beeinträchtigung bei Morbus Parkinson ist.

8. Die Verbindung oder das pharmazeutisch verträgliche Salz davon zur Verwendung nach Anspruch 6, wobei die Lewy-Körperchen-Krankheit diffuse Lewy-Körperchen-Krankheit ist.

9. Die Verbindung oder das pharmazeutisch verträgliche Salz davon zur Verwendung nach Anspruch 6, wobei die Lewy-Körperchen-Krankheit Demenz mit Lewy-Körperchen ist.

10. Die Verbindung oder das pharmazeutisch verträgliche Salz davon zur Verwendung nach Anspruch 6, wobei die Lewy-Körperchen-Krankheit eine mit Lewy-Körperchen-Krankheit in Zusammenhang stehende Bewegungsstörung ist.

## Revendications

1. Composé représenté par la formule (I) :

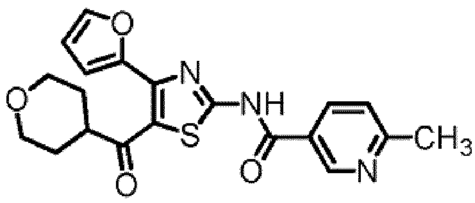

(I)

ou un sel pharmaceutiquement acceptable de celui-ci, pour une utilisation dans le traitement et/ou la prophylaxie d'un dysfonctionnement du lobe frontal, dans lequel le dysfonctionnement du lobe frontal est

(1) un trouble cognitif choisi dans le groupe constitué par un trouble cognitif associé à la maladie de Parkinson, un trouble cognitif lié à un stress chronique, une démence à corps de Lewy et une démence fronto-temporale ; ou
(2) un trouble cognitif dû à un déclin de la fonction de la dopamine dans le cortex préfrontal médial.

2. Composé ou sel pharmaceutiquement acceptable de celui-ci pour une utilisation selon la revendication 1, dans lequel le trouble cognitif est un trouble cognitif associé à la maladie de Parkinson.

3. Composé ou sel pharmaceutiquement acceptable de celui-ci pour une utilisation selon la revendication 2, dans lequel le trouble cognitif associé à la maladie de Parkinson est un dysfonctionnement exécutif, un trouble de la mémoire, une détérioration de la cognition visuo-spatiale ou une perturbation de l'odorat.

4. Composé ou sel pharmaceutiquement acceptable de celui-ci pour une utilisation selon la revendication 1, dans lequel le trouble cognitif est un trouble cognitif lié à un stress chronique.

5. Composé ou sel pharmaceutiquement acceptable de celui-ci pour une utilisation selon la revendication 1, dans lequel le trouble cognitif est une démence à corps de Lewy.

**6.** Composé représenté par la formule (I) :

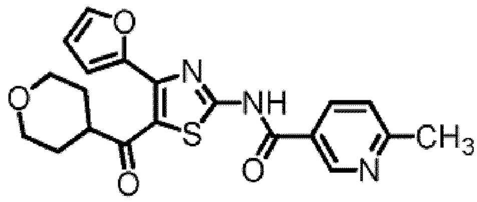

(I)

ou un sel pharmaceutiquement acceptable de celui-ci, pour une utilisation dans le traitement et/ou la prophylaxie d'une maladie à corps de Lewy.

**7.** Composé ou sel pharmaceutiquement acceptable de celui-ci pour une utilisation selon la revendication 6, dans lequel la maladie à corps de Lewy est un trouble cognitif associé à la maladie de Parkinson.

**8.** Composé ou sel pharmaceutiquement acceptable de celui-ci pour une utilisation selon la revendication 6, dans lequel la maladie à corps de Lewy est une maladie à corps de Lewy diffus.

**9.** Composé ou sel pharmaceutiquement acceptable de celui-ci pour une utilisation selon la revendication 6, dans lequel la maladie à corps de Lewy est une démence à corps de Lewy.

**10.** Composé ou sel pharmaceutiquement acceptable de celui-ci pour une utilisation selon la revendication 6, dans lequel la maladie à corps de Lewy est un trouble du mouvement associé à une maladie à corps de Lewy.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9912546 A
- WO 2005063743 A
- WO 2010126082 A
- WO 2012060844 A1

### Non-patent literature cited in the description


- *American Journal of Psychiatry,* 1998, vol. 155 (3), 344
- *Archives of Neurology,* 2000, vol. 57, 470
- *The Journal of Neuroscience,* 2000, vol. 20 (4), 1568
- *Neurology,* 2010, vol. 74, 885
- *Journal of Chemical Neuroanatomy,* 2011, vol. 42, 242
- *Brain,* 2014, vol. 137, 2493
- *Brain Research,* 2009, vol. 1303, 74
- *Behavioural Brain Research,* 2005, vol. 159, 197
- *Psychopharmacology,* 2013, vol. 230, 345
- *Behavioural Brain Research,* 1988, vol. 31, 47
- *Science,* 2012, vol. 338, 949
- *Behavioral Brain Reexplore,* 2010, vol. 208, 274
- *Neuroscience and Biobehavioral Reviews,* 2004, vol. 28, 497
- *Journal of Biomedical Science,* 2010, vol. 17, 9